# EUROPEAN PATENT APPLICATION

(11) **EP 2 774 611 A1**
(43) Date of publication of application: **10.09.2014**
(21) Application number: 12838261.1
(22) Date of filing: 06.09.2012
(51) Int. Cl.: A61K 31/295, A61K 9/10, A61P 35/00, B22F 9/00

(54) **MAGNETIC COMPOSITION, AND METHOD FOR PRODUCING SAME**

(30) Priority: 06.10.2011 JP 2011222354
(71) Applicant: IHI Corporation, Tokyo 135-8710 (JP); Ishikawa, Yoshihiro, Tokyo 160-0022 (JP)
(72) Inventor: ISHIKAWA, Yoshihiro, Tokyo 160-0022 (JP); EGUCHI, Haruki, Tokyo 135-8710 (JP)
(74) Representative: Smith, Matthew
(86) International application number: PCT/JP2012/072794
(87) International publication number: WO 2013/051363

(57) **Abstract**

A magnetic composition containing a metal-salen complex compound which can be securely guided by a magnetic field to a target area to be preferably treated, and a method for producing the magnetic composition are provided. The magnetic composition is prepared by dispersing magnetic particles, which are obtained by coating a metal-salen complex compound with a dispersant, in a polar solvent by means of the dispersant. Furthermore, the magnetic composition production method includes a first step of mixing the metal-salen complex compound with the dispersant in an organic solvent and coating the metal-salen complex compound with the dispersant and a second step of dispersing the metal-salen complex in a polar solvent.

## Description

### [Technical Field]

The present invention relates to a magnetic composition, in which particulates of a magnetic meta-salen complex compound coated with a dispersant is dispersed in a solvent, and a method for producing such a magnetic composition.

### [Background Art]

Conventionally, a metal-salen complex compound has been known as a magnetic organic compound (International Publication WO2010/058520). The metal-salen complex compound can be guide to a target affected site tissue and be aggregated at the affected site tissue by applying the metal-salen complex compound to a human or an animal and then applying a magnetic field to this metal-salen complex compound externally. As a result, pharmacological effects of the metal-salen complex compound can be aggregated and exhibited at the target affected site tissue. For example, an anticancer action is known as the pharmacological effects of the metal-salen complex compound. Moreover, the above-mentioned international publication also describes that medical molecules can be guided to a target area by means of an external magnetic field by combining the medical molecules with the metal-salen complex compound. In other words, the metal-salen complex compound also serves as a carrier of the medical molecules.

Furthermore, a review article about an organic magnetic substance is introduced and descries that a magnet is produced with polymeric materials by means of synthesis of "high-spin molecules" having more parallel spins than those of conventional metallic magnetic substances (for example, see Non Patent Literature 1).

Furthermore, a technique that replaces platinum contained in cisplatin with another element is also introduced (for example, see Non Patent Literature 2).

### [Citation List]

### [Patent Literature]

[Patent Literature 1] International Publication WO2010/058520
[Non Patent Literature 1] Hiizu Iwamura, "Molecular Design Aimed at Organic Ferromagnetic Substances," Feb. 1989 issue, p.p. 76-88
[Non Patent Literature 2] Krsity Cochran et al., Structural Chemistry, 13 (2002), p.p. 133-140

### [Summary of Invention]

### [Problems to be Solved by the Invention]

After reviewing the matter, the inventor of this application has found a problem of the inability to securely guide a metal-salen compound to an area where a magnetic field is applied even if an injection of the metal-salen complex compound is applied to an animal and the magnetic field is then applied to the animal.

Moreover, Non Patent Literature 1 or Non Patent Literature 2 does not refer to magnetization of the drug itself.

So, it is an object of the present invention to provide a magnetic composition, which can be securely guided to a target area to be preferably treated by means of a magnetic field, and a method for producing this magnetic composition.

### [Solution to Problem]

In order to achieve this object, the present invention is characterized in that it is a magnetic composition prepared by dispersing magnetic particles, which are a metal-salen complex compound coated with a dispersant, in a polar solvent by means of the dispersant.

As a result of thorough examination, the inventor of this application has found that dispersibility of the metal-salen complex compound in a solvent for injections and transfusions is not sufficient; and if a magnetic field is applied to the solvent for injections or transfusions, in which the metal-salen complex compound is dispersed, a phenomenon in which the metal-salen complex compound agglomerates will occur. Even if the magnetic field is applied towards the target area, the magnetic field will naturally have an influence on the periphery of the target area. So, the metal-salen complex compound will agglomerate in the peripheral area due to the influence of the magnetic field before reaching the target area and the metal-salen complex compound may not be able to move within fine capillaries (5 to 10µm) from there to the target area.

Since the magnetic composition according to the present invention is prepared by dispersing magnetic particles, which are coated by a dispersant, in a polar solver by means of the dispersant, the metal-salen complex compound can be sufficiently dispersed in the polar solvent. Therefore, even if the metal-salen complex compound is exposed to the magnetic field in the peripheral area of the target area (target lesion area), it will securely reach the target area without agglomerating.

Moreover, the metal-salen complex compound contained in the magnetic composition according to the present invention can exhibit non-agglomerating property that will not agglomerate within capillaries under a magnetic field environment. Accordingly, for example, even if the magnetic field is applied after introducing, for example, the solvent for injections or transfusions, in which the magnetic composition according to the present invention is dispersed, into the body, it is possible to securely prevent agglomeration of the metal-salen complex compound. Therefore, the metal-salen complex compound can move within the fine capillaries (5 to 10µm), so that it is possible to make the metal-salen complex compound reach the target area more securely.

Furthermore, the present invention provides a magnetic composition production method including a first step of mixing a metal-salen complex compound with a dispersant in an organic solvent and coating the metal-salen complex compound with the dispersant and a second step of dispersing the metal-salen complex in a polar solvent.

By this production method, it is possible to produce the magnetic composition by dispersing magnetic particles, which are prepared by coating the metal-salen complex compound with the dispersant, in the polar solvent by means of the dispersant.

### [Advantageous Effects of Invention]

According to the present invention, it is possible to provide a magnetic composition containing a metal-salen complex compound, which can be securely guided by a magnetic field to a target area to be preferably treated, and a method for producing the magnetic composition.

### [Description of Embodiments]

The metal-salen complex compound which is applied to this invention is a structure, in which a salen ligand (N,N'-bis(salicylidene) ethylenediamine) coordinates with metal, or its derivatives. A specific example of the metal-salen complex compound is explained in the aforementioned International Publication WO2010/058520. Compounds obtained by combining functional molecules such as medical molecules with the metal-salen complex structure are included in the metal-salen complex of the present invention.

The step of coating the metal-salen complex compound with the dispersant is executed in an organic solvent by using a dispersant having an affinity to the organic solvent. Then, when the metal-salen complex compound coated with the dispersant is separated and the metal-salen complex compound is introduced into the polar solvent, the dispersant makes the metal-salen complex compound dispersed in the polar solvent. A binding form based on the van der Waals' interaction and electrostatic interaction is formed between the metal-salen complex compound and the dispersant.

According to a preferred embodiment, a polar group of the dispersant should preferably be protected by a protective group. As the protective group which protects the polar group of the dispersant is desorbed by the polar solvent, the polar group enters into an ionized state and the polar group of the dispersant disperses the metal-salen complex compound in the polar solvent such as a physiological saline.

As the metal-salen complex compound is dispersed in the polar solvent, the metal-salen complex compound becomes nanoparticles whose average particle diameter should preferably be 10 nm or more and 500 nm or less; and when the solvent for injections or transfusions or the like to which these nanoparticles are added is introduced into the body, agglomeration of the metal-salen complex compound can be prevented even if the magnetic field is applied within the capillaries inside the body (in other words, non-agglomerating property is exhibited).

Examples of the dispersant used to coat the metal-salen complex compound are not particularly limited as long as the dispersant can disperse the metal-salen complex compound in the polar solvent such as the physiological saline; however, a dispersant for metal nanoparticles is preferred. Examples of this type of dispersant are described in, for example, Japanese Patent Application Laid-Open (Kokai) Publication No. 2011-68988 and Japanese Patent Application Laid-Open (Kokai) Publication No. 2008-127241.

If the magnetic field applied to the metal-salen complex compound is strong, there is a possibility that the metal-salen complex compound may agglomerate. On the other hand, if magnetic field intensity is low, there is a possibility that the metal-salen complex compound may not be guided to the target area. From this point of view, a preferred range of magnetic field intensity is from 0.3 T to 1 T. Furthermore, a preferred range of percentage content of the metal-salen complex compound in the magnetic composition is 10% or more and 60% or less.

### [Examples]

### [Example of Production of Iron-Salen Complex Compound]

Next, an iron-salen complex having an amino group as a substituent was produced as explained below.

### Step 1:

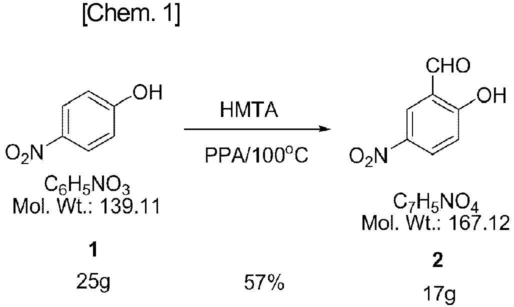

A mixture of 4-nitrophenol (25 g, 0.18 mol), hexamethylene tetramine (25 g, 0.18 mol), and polyphosphoric acid (200 ml) was agitated for 1 hour at 100°C. Subsequently, the mixture was introduced into 500 ml of ethyl acetate and 1 L of water, and agitated until the mixture became completely dissolved. In addition, as a result of additionally adding 400 ml of ethyl acetate to the foregoing solution, the solution separated into two phases. An aqueous phase was removed from the two phases and the remaining compound was washed twice with a saline solvent; and as a result of drying with anhydrous MgSO₄, 17 g of Compound 2 (yield 57%) was synthesized.

### Step 2:

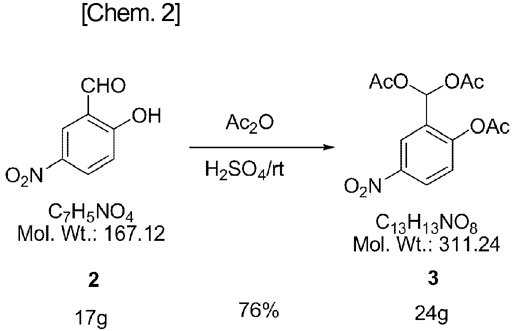

Next, Compound 2 (17 g, 0.10 mol), acetic anhydride (200 ml), and sulfuric acid (15 mL) were agitated for 1 hour at room temperature. The obtained solution was mixed in ice water (2 L) for 0.5 hours, and hydrolysis was performed. When the obtained solution was filtered and dried in the atmosphere, a white powdery substance was obtained. As a result of recrystallizing the powder with a solution containing ethyl acetate, 24 g of compound 3 (yield 76%) was obtained as white crystals.

### Step 3:

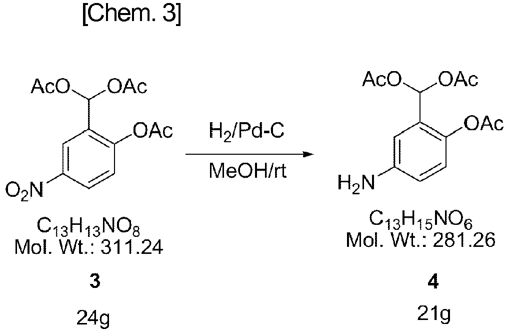

Subsequently, a mixture of carbon (2.4 g) carrying 10% palladium was reduced to Compound 3 (24 g, 77 mmol) and methanol (500 ml) in a hydrogen reduction atmosphere overnight at 1.5 atmospheric pressure. After completion, the product was filtered with a filter and brown oily Compound 4 (21 g) was synthesized.

### Step 4, 5:

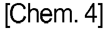

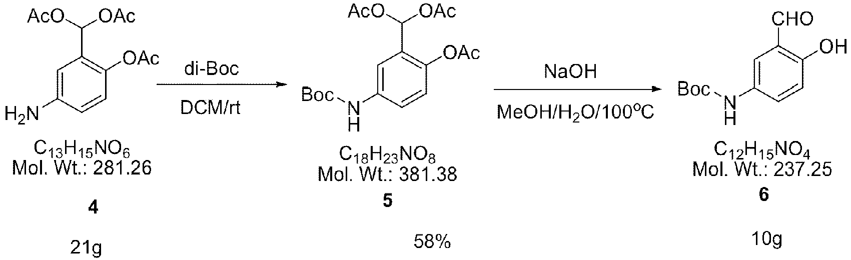

Next, compound 4 (21 g, 75 mmol) and di(tert-butyl) dicarbonate (18 g, 82 mmol) were introduced into dichloromethane (DCM) (200 ml) and the obtained mixture was agitated overnight in a nitrogen atmosphere. The obtained solution was vaporized in a vacuum and then dissolved in methanol (100 ml). Subsequently, sodium hydroxide (15 g, 374 mmol) and water (50 ml) were added and refluxed for 5 hours. Then, the product was cooled, filtered with a filter, washed with water, and dried in a vacuum, and a brown compound was thereby obtained. The obtained compound was twice subject to flash chromatography using silica gel, thereby obtaining 10 g of Compound 6 (yield 58%).

### Step 6:

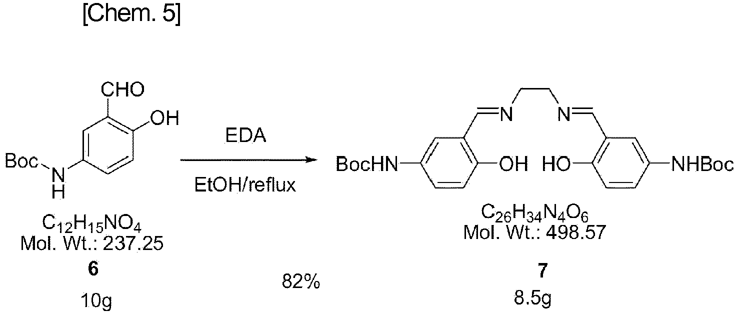

Compound 6 (10 g, 42 mmol) was introduced into 400 ml of dehydrated ethanol, refluxed while being heated, and several drops of ethylenediamine (1.3 g, 21 mmol) was added to 20 ml of dehydrated ethanol and agitated for 0.5 hours. The mixed solution was cooled in a vessel made of ice and mixed for 15 minutes. Subsequently, the product was washed with 200 ml of ethanol and filtered and dried in a vacuum, thereby synthesizing 8.5 g of Compound 7 (yield 82%).

### Step 7:

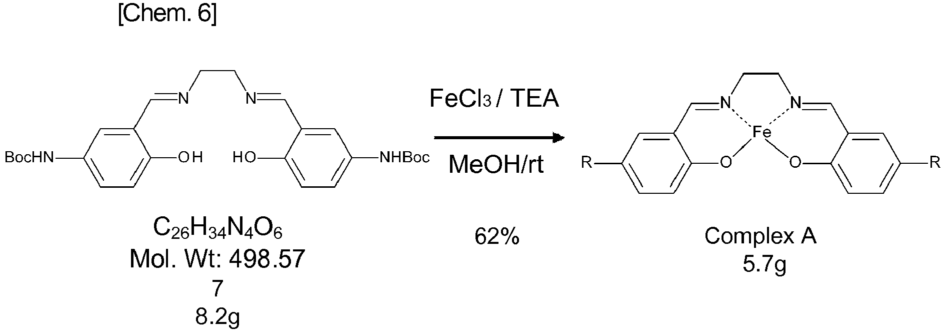

Compound 7 (8.2 g, 16 mmol) and triethylamine (22 ml, 160 mmol) were introduced into dehydrated methanol (50 mL), and a solution obtained by adding FeCl₃ (2.7 g, 16 mmol) to 10 ml of methanol was mixed under a nitrogen atmosphere. As a result of mixing the product at room temperature under a nitrogen atmosphere, a brown compound was obtained. The product was then dried in a vacuum. The obtained compound was diluted in 400 ml of dichloromethane, washed twice with a saline solution, dried in a vacuum, and an iron-salen complex compound (complex A; however, R=H) was obtained.

### [Production Example of Cobalt-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding CoCl₂ (Cobalt (II) chloride by Alfa Aesar) (2.7 g, 16 mmol) to methanol (10mL) was mixed under a nitrogen atmosphere. Then, a cobalt-salen complex compound was obtained by the same method as the method for producing the iron-salen complex compound.

### [Production Example of Nickel-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding NiCl₂ (Nickel (II) chloride by Alfa Aesar) (2.7 g, 16 mmol) to methanol (10mL) was mixed under a nitrogen atmosphere. Then, a nickel-salen complex compound was obtained by the same method as the method for producing the iron-salen complex compound.

### [Production Example of Molybdenum-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding MoCl₃ (Molybdenum (III) chloride by Alfa Aesar) (2.7 g, 16 mmol) to methanol (10mL) was mixed under a nitrogen atmosphere. Then, a molybdenum-salen complex compound was obtained by the same method as the method for producing the iron-salen complex compound.

### [Production Example of Ruthenium-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding RuCl₃ (Ruthenium (III) chloride by Alfa Aesar) (2.7 g, 16 mmol) to methanol (10mL) was mixed under a nitrogen atmosphere. Then, a ruthenium-salen complex compound was obtained by the same method as the method for producing the iron-salen complex compound.

### [Production Example of Rhodium-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding RhCl₃ (Rhodium (III) chloride by Alfa Aesar) (2.7 g, 16 mmol) to methanol (10mL) was mixed under a nitrogen atmosphere. Then, a rhodium-salen complex compound was obtained by the same method as the method for producing the iron-salen complex compound.

### [Production Example of Palladium-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding PdCl₂ (Palladium (II) chloride by Alfa Aesar) (2.7 g, 16 mmol) to methanol (10mL) was mixed under a nitrogen atmosphere. Then, a palladium-salen complex compound was obtained by the same method as the method for producing the iron-salen complex compound.

### [Production Example of Tungsten-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding WCl₆ (Tungsten (VI) chloride by Alfa Aesar) (2.7 g, 16 mmol) to methanol (10mL) was mixed under a nitrogen atmosphere. Then, a tungsten-salen complex compound was obtained by the same method as the method for producing the iron-salen complex compound.

### [Production Example of Rhenium-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding ReCl₅ (Rhenium (V) chloride by Alfa Aesar) (2.7 g, 16 mmol) to methanol (10mL) was mixed under a nitrogen atmosphere. Then, a rhenium-salen complex compound was obtained by the same method as the method for producing the iron-salen complex compound.

### [Production Example of Osmium-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding osmium-salen trihydrate (Osmium (III) chloride trihydrate by Alfa Aesar) (2.7 g, 16 mmol) to methanol (1 0mL) was mixed under a nitrogen atmosphere. Then, an osmium-salen complex compound was obtained by the same method as the method for producing the iron-salen complex compound.

### [Production Example of Iridium-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding IrCl₃ (Iridium (III) chloride by Alfa Aesar) (2.7 g, 16 mmol) to methanol (10mL) was mixed under a nitrogen atmosphere. Then, an iridium-salen complex compound was obtained by the same method as the method for producing the iron-salen complex compound.

### [Production Example of Platinum-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding PtCl₂ (Platinum (II) chloride by Alfa Aesar) (2.7 g, 16 mmol) to methanol (10mL) was mixed under a nitrogen atmosphere. Then, a platinum-salen complex compound was obtained by the same method as the method for producing the iron-salen complex compound.

### [Production Example of Neodymium-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding NdCl₃ (Neodymium (III) chloride by Alfa Aesar) (2.7 g, 16 mmol) to methanol (10mL) was mixed under a nitrogen atmosphere. Then, a neodymium-salen complex compound was obtained by the same method as the method for producing the iron-salen complex compound.

### [Production Example of Samarium-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding SmCl₃ (Samarium (III) chloride by Alfa Aesar) (2.7 g, 16 mmol) to methanol (10mL) was mixed under a nitrogen atmosphere. Then, a samarium-salen complex compound was obtained by the same method as the method for producing the iron-salen complex compound.

### [Production Example of Europium-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding EuCl₃ (Europium (III) chloride by Alfa Aesar) (2.7 g, 16 mmol) to methanol (10mL) was mixed under a nitrogen atmosphere. Then, a europium-salen complex compound was obtained by the same method as the method for producing the iron-salen complex compound.

### [Production Example of Gadolinium-Salen Complex Compound]

After producing Compound 7 by the aforementioned method, Compound 7 (8.2 g, 16 mmol) and triethylamine (22 mL, 160 mmol) were introduced into dehydrated methanol (50 mL), and a solution obtained by adding GdCl₃ (Gadolinium (III) chloride by Alfa Aesar) (2.7 g, 16 mmol) to methanol (10mL) was mixed under a nitrogen atmosphere. Then, a gadolinium-salen complex compound was obtained by the same method as the method for producing the iron-salen complex compound.

Next, production examples (Examples) of a magnetic nanoparticle fluid dispersion in which the iron-salen complex compound is dispersed will be explained. Firstly, a case where a dispersant whose polar group is protected by a protective group is used will be explained.

### [Example of Case Where Protective Group Changes from Boc Protection to Amine System]

A DMSO solution (1 M; and volume: 1 mL) of (Boc-aminooxy) acetic acid (Fluka rp.), which was a dispersant, was added to the iron-salen complex (approximately 10 mg/mL) which was suspended in 10 mL of chloroform; and the obtained solution was vigorously agitated for 3 to 5 hours at room temperature, thereby obtaining magnetic nanoparticles which have chloroform dispersibility. Then, 10 mL of 1 N hydrogen chloride solution was added and the obtained mixture was agitated vigorously in a two phase system. At that time, the magnetic nanoparticles moved to an aqueous phase, thereby obtaining an aqueous magnetic nanoparticle solution having aqueous dispersibility. Under this circumstance, the pH of the aqueous phase was made to be 2 or less. (Boc-aminooxy) acetic acid ([(tert-butoxycarbonyl) amino-oxy] acetic acid) is the protective group described earlier and the DMSO is the dispersant.

Even when a magnet (magnetic field intensity: approximately 0.5 T) was placed closer to the thus-obtained magnetic nanoparticle fluid dispersion, agglomeration of the particles was not observed. Furthermore, a particle distribution was checked with a transmission electron microscope, an average particle diameter was approximately 80 nm.

### [Example of Case Where Protective Group Changes from Fmoc Deprotection to Amine System]

A DMSO solution (concentration: 1 M; and volume: 1 mL) of Fmoc-Asp-OH (WATANABE CHEMICAL), which was a dispersant, was added to the iron-salen complex (approximately 10 mg/mL) which was suspended in 10 mL of chloroform; and the obtained solution was vigorously agitated for 3 to 5 hours at room temperature, thereby obtaining magnetic nanoparticles which have chloroform dispersibility. Then, 10 mL of 1 N aqueous sodium hydroxide was added and the obtained mixture was agitated vigorously in a two phase system. At that time, the magnetic nanoparticles moved to an aqueous phase, thereby obtaining an aqueous magnetic nanoparticle solution having aqueous dispersibility. Under this circumstance, the pH of the aqueous phase was made to be 8 or more. Even when the magnet (magnetic field intensity: approximately 0.5 T) was placed closer to the thus-obtained magnetic nanoparticle fluid dispersion, agglomeration of the particles was not observed. Furthermore, a particle distribution was checked with the transmission electron microscope, an average particle diameter was approximately 70 nm. Fmoc-Asp-OH(N-[(9H-fluorene-9- ylmethoxy) carbonyl]-(L)-aspartic acid)is the protective group described earlier.

### [Example of Case Where Protective Group Changes from Ethyl Ester to Carboxyhydrazine]

A DMSO solution (concentration: 1 M; and volume: 1 mL) of monoethyl adipate (Tokyo Chemical Industry Co., Ltd.), which was a dispersant, was added to the iron-salen complex (approximately 10 mg/mL) which was suspended in 10 mL of chloroform; and the obtained solution was vigorously agitated for 3 to 5 hours at room temperature, thereby obtaining magnetic nanoparticles which have chloroform dispersibility. Then, 10 mL of hydrazine monohydrate was added and the obtained mixture was agitated vigorously. At that time, precipitates were produced in the chloroform solution. A supermatant liquid was thrown away once and distilled water was added, thereby obtaining an aqueous magnetic nanoparticle solution having aqueous dispersibility. At that time, the pH of the aqueous phase was made to be 3 or less. Even when the magnet (magnetic field intensity: approximately 0.5 T) was placed closer to the thus-obtained magnetic nanoparticle fluid dispersion, agglomeration of the particles was not observed. Furthermore, a particle distribution was checked with the transmission electron microscope, an average particle diameter was approximately 90 nm. The monoethyl adipate is the protective group described earlier.

### [Example of Case Where Protective Group Changes from Ethyl Ester to Carboxylic Acid]

A DMSO solution (concentration: 1 M; and volume: 1 mL) of monoethyl adipate (Tokyo Chemical Industry Co., Ltd.), which was a dispersant, was added to the iron-salen complex (approximately 10 mg/mL) which was suspended in 10 mL of chloroform; and the obtained solution was vigorously agitated for 3 to 5 hours at room temperature, thereby obtaining magnetic nanoparticles which have chloroform dispersibility. Then, 10 mL of 1 N aqueous sodium hydroxide was added and the obtained mixture was agitated vigorously. At that time, the magnetic nanoparticles moved to an aqueous phase, thereby obtaining an aqueous magnetic nanoparticle solution having aqueous dispersibility. At that time, the pH of the aqueous phase was made to be 8 or more. Even when the magnet (magnetic field intensity: approximately 0.5 T) was placed closer to the thus-obtained magnetic nanoparticle fluid dispersion, agglomeration of the particles was not observed. Furthermore, a particle distribution was checked with the transmission electron microscope, an average particle diameter was approximately 80 nm.

Next, metal-salen complex compounds other than the iron-salen complex compound (the respective metal-salen complex compounds produced in Examples above) were used as the metal-salen complex compound and magnetic nanoparticle fluid dispersions were produced respectively according to Examples. Subsequently, when the magnet (magnetic field intensity: approximately 0.5 T) was placed closer to each of the magnetic nanoparticle fluid dispersions, agglomeration of the particles was not observed. Furthermore, a particle distribution was checked with the transmission electron microscope, an average particle diameter was approximately 100 to 600 nm.

## Claims

1. A magnetic composition prepared by dispersing magnetic particles, which are obtained by coating a metal-salen complex compound coated with a dispersant, in a polar solvent by means of the dispersant.

2. The magnetic composition according to claim 1, wherein the metal-salen complex compound exhibits non-agglomerating property within capillaries under a magnetic field environment.

3. The magnetic composition according to claim 2, wherein magnetic field intensity under the magnetic field environment is 0.3 T to 1 T.

4. The magnetic composition according to claim 1 or 2, wherein a particle diameter of the metal-salen complex compound is 10 nm or more and 500 nm or less.

5. The magnetic composition according to any one of claims 1 to 3, wherein a polar group of the dispersant is protected by a protective group.

6. The magnetic composition according to any one of claims 1 to 5, wherein the metal-salen complex compound is contained at 10 wt% or more and 60 wt% or less.

7. A magnetic composition production method comprising:
a first step of mixing a metal-salen complex compound with a dispersant in an organic solvent and coating the metal-salen complex compound with the dispersant; and
a second step of dispersing the metal-salen complex in a polar solvent.

8. The magnetic composition production method according to claim 7, wherein magnetic particles which are made of the metal-salen complex compound coated with the dispersant are introduced into the polar solvent, a protective group is desorbed from a polar group of the dispersant, and the magnetic particles are dispersed in the polar solvent.
